(19) Europäisches Patentamt / European Patent Office / Office européen des brevets

(11) EP 4 056 550 A1

(12) **EUROPÄISCHE PATENTANMELDUNG**

(43) Veröffentlichungstag:
**14.09.2022 Patentblatt 2022/37**

(21) Anmeldenummer: **21162203.0**

(22) Anmeldetag: **12.03.2021**

(51) Internationale Patentklassifikation (IPC):
***C07C 41/60*** *(2006.01)* ***C07C 43/32*** *(2006.01)*

(52) Gemeinsame Patentklassifikation (CPC):
(C-Sets verfügbar)
**C07C 41/60** (Forts.)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Clariant International Ltd**
**4132 Muttenz (CH)**

(72) Erfinder:
- **ENDRES, Andreas**
**89264 Weißenhorn (DE)**
- **HÖVELMANN, Felix**
**84453 Mühldorf (DE)**

(74) Vertreter: **Mikulecky, Klaus**
**Clariant Produkte (Deutschland) GmbH**
**Patent & License Management**
**Industriepark Höchst, G 860**
**65926 Frankfurt am Main (DE)**

(54) **VERFAHREN ZUR HERSTELLUNG VON ORTHOESTERN**

(57) Die vorliegende Erfindung betrifft ein Verfahren zur Herstellung eines Orthoesters der Formel (I)

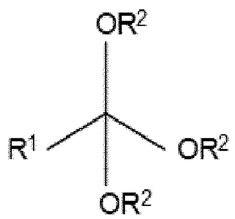

(I)

worin
$R^1$
für H oder einen $C_1$- bis $C_8$-Alkylrest,
$R^2$
für einen $C_1$- bis $C_6$-Alkylrest, oder eine Gruppe der Formel (IV)

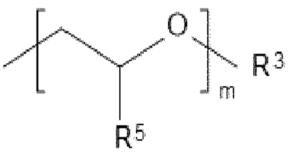

(IV)

$R^3$
für einen $C_1$- bis $C_6$-Alkylrest, und
m
für eine Zahl von 1 bis 40 stehen, mit der Maßgabe, dass mindestens einer der Reste $R^2$ für eine Gruppe der Formel (IV), und
$R^5$
für H oder Methyl steht
indem mindestens ein Alkohol der Formel (II)

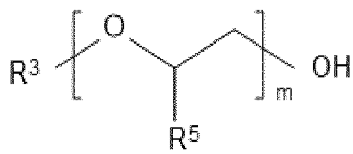

(II)

mit einem Orthoester der Formel (III)

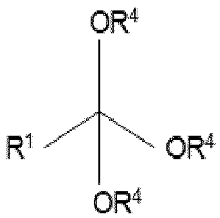

(III)

worin $R^4$ für eine $C_1$- bis $C_6$-Alkylgruppe steht
bei einer Temperatur von 60 bis 280°C umgesetzt wird, und die dabei entstehenden Alkohole $R^4$-OH destillativ entfernt werden, dadurch
gekennzeichnet, dass die Umsetzung entweder in Abwesenheit eines Katalysators erfolgt, eine aromatische Carbonsäure oder ein organischer Phosphorsäureester als Katalysator zugegen ist.

EP 4 056 550 A1

(52) Gemeinsame Patentklassifikation (CPC): (Forts.)

C-Sets
**C07C 41/60, C07C 43/32**

## Beschreibung

Die vorliegende Erfindung betrifft ein neues Verfahren zur Herstellung von alkoxylierten Orthoestern aus alkoxylierten Alkoholen und Trialkyl-Orthoestern, das in Abwesenheit von anorganischen Bestandteilen durchgeführt wird.

Stand der Technik

Für die Herstellung von Orthocarbonsäureester (im Folgenden auch Orthoester genannt) sind verschiedene Verfahren bekannt.

US-3419580 beschreibt die Herstellung von Orthoestern durch Reaktion von beispielsweise Vinylidenchlorid mit Natriumsalzen von Diethylenglykolmonomethylether, in Anlehnung an die Williamson-Ethersynthese.

GB-1128963 beschreibt unter Bezug auf ältere Literatur die Reaktion von Nitrilen und Chlorwasserstoffsäure mit absolutem Alkohol zum Imidoesterhydrochlorid als Zwischenprodukt, das mit weiterem Alkohol in Anwesenheit einer Base zur Bildung eines Orthoesters und Ammoniumchlorid führt. Die Reaktion wird normalerweise in einem Lösungsmittel wie Chlorbenzol, Diäthyläther, Chloroform, o-Dichlorbenzol oder Petroläther durchgeführt, aus welchem das Ammoniumchlorid ausfällt. In einer Abwandlung dieser Reaktion nach GB-1128963 werden der Alkohol und die Chlorwasserstoffsäure mit einer Nitril- oder einer Dinitril-Verbindung zur Reaktion gebracht, wobei z. B. mit Adipo-1,6-dinitril ein Orthoadipat entsteht.

DE-2062034 beschreibt die Herstellung von Orthoestern aus u. a. Trialkylorthoformiaten und Glykolethern unter Verwendung von Lewis Säuren wie z. B. Aluminiumtrichlorid. Dieses Verfahren liefert nur geringe Ausbeute, erfordert die Verwendung von Lewis-Säuren und eine aufwändige Reinigung bzw. Destillation der Produkte.

R. M. Roberts, T. D. Higgins, P. R. Noyes, J. Am. Chem. Soc. 1955 (77), 3801, und E. R. Alexander, H. M. Busch, J. Am. Chem. Soc. 1952 (74), 554 lehren die Umesterung von kurzkettigen Trialkylorthoformiaten und -acetaten mit komplexeren Alkoholen bei Anwesenheit von Mineralsäuren.

US-2867667 lehrt die Reaktion von Trialkylorthoestern mit Alkylenoxyden in Anwesenheit von $BF_3$, wobei man Tris-glykolätherorthoester oder Mischungen aus Glykolethern und Alkylorthoester erhält.

All diese Verfahren verwenden anorganische Substanzen oder Salze bzw. Halogenide, welche entweder im Produkt verbleiben oder aufwändig entfernt werden müssen. Durch die Aufarbeitung der Produkte wird zusätzlich die isolierte Ausbeute deutlich reduziert.

Es war somit die Aufgabe der vorliegenden Erfindung, ein Verfahren zu entwickeln, das es ermöglicht Orthoester, bevorzugt Orthoformiat oder -acetat, herzustellen ohne anorganische Katalysatoren und Nebenprodukte entfernen zu müssen. Es soll möglichst keine Aufarbeitung der Produkte erforderlich sein, die die Ausbeute an Orthoester mindert. Das Produkt soll möglichst klar und homogen anfallen, und es sollen keine Reste von unlöslichen bzw. anorganischen Katalysatoren enthalten sein.

Gegenstand der Erfindung ist ein Verfahren zur Herstellung eines Orthoesters der Formel (I)

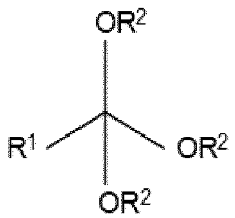

(I)

worin

$R^1$ für H oder einen $C_1$- bis $C_8$-Alkylrest,
$R^2$ für einen $C_1$- bis $C_6$-Alkylrest, oder eine Gruppe der Formel (IV)

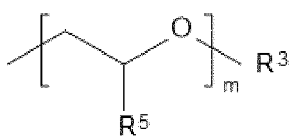

(IV)

$R^3$ für einen $C_1$- bis $C_6$-Alkylrest,
$R^5$ für H oder Methyl, und
m für eine Zahl von 1 bis 40 stehen,

mit der Maßgabe, dass mindestens einer der Reste $R^2$ für eine Gruppe der Formel (IV) steht, indem mindestens ein Alkohol der Formel (II)

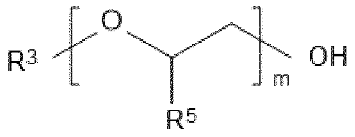

(II)

mit einem Orthoester der Formel (III)

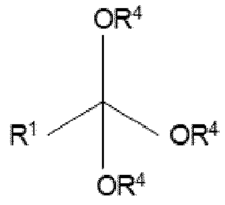

(III)

worin $R^4$ für eine $C_1$- bis $C_6$-Alkylgruppe steht,
bei einer Temperatur von 60 bis 280°C umgesetzt wird, und die dabei entstehenden Alkohole $R^4$-OH destillativ entfernt werden, dadurch gekennzeichnet, dass die Umsetzung entweder in Abwesenheit eines Katalysators erfolgt oder ein Katalysator, ausgewählt aus aromatischen Carbonsäuren und organischen Phosphorsäureestern zugegen ist.

Wird einer der drei Reste $R^4O$ des Orthoesters der Formel (III) mit dem Alkohol der Formel (II) umgesetzt, so entspricht das erhaltene Produkt der Formel (I) worin $R^2$ einmal der Formel (IV) entspricht, und zweimal einer Alkylgruppe. Werden zwei der drei Reste $R^4O$ des Orthoesters der Formel (III) mit dem Alkohol der Formel (II) umgesetzt, so entspricht das erhaltene Produkt der Formel (I) worin $R^2$ zweimal der Formel (IV) entspricht, und einmal einer Alkylgruppe. Werden alle Reste $R^4O$ des Orthoesters der Formel (III) mit dem Alkohol der Formel (II) umgesetzt, so entsprechen alle Reste $R^2$ der Formel (IV). Es sind also folgende Reaktionsprodukte der Formel (I) möglich:

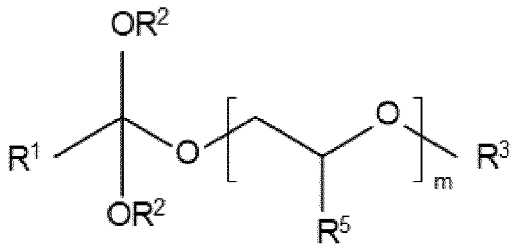

(Ia)

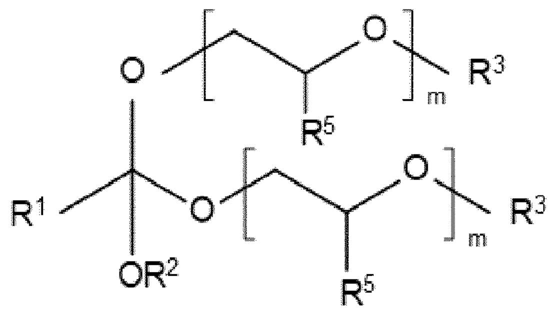

(Ib)

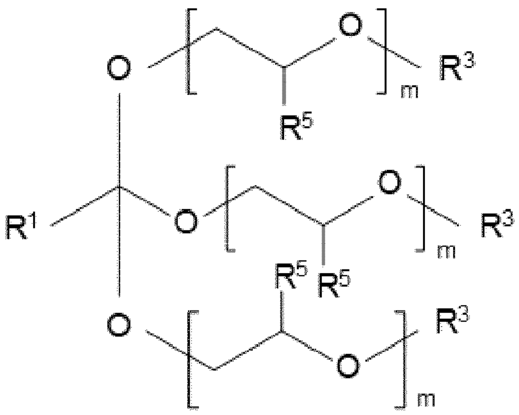

(Ic)

Es ist auch möglich, eine Mischung aus zwei, drei oder mehr Alkoholen der Formel (II) für die Reaktion einzusetzen, die sich in $R^3$, $R^5$ und/oder m unterscheiden. Dies bedeutet, dass die Reste der Formel (II) in den Verbindungen der Formel (I) voneinander unterschiedlich sein können.

In einer bevorzugten Ausführungsform beträgt das molare Verhältnis von Alkohol der Formel (I zu Orthoester der Formel (III) mindestens 2 zu 1, mehr bevorzugt mindestens 3 zu 1.

In einer bevorzugten Ausführungsform wird das erfindungsgemäße Verfahren zur Herstellung eines Orthoesters unter Verwendung eines Katalysators durchgeführt. Der Katalysator ist dann eine organische Carbonsäure oder ein organischer Phosphorsäureester, welche im Endprodukt löslich sind. Besonders bevorzugt als Katalysator sind Benzoesäure und 2-Ethylhexanolphosphat.

In einer bevorzugten Ausführungsform werden die während der Reaktion gebildeten Alkohole $R^4OH$ über eine Kolonne destillativ abgetrennt. In einer bevorzugten Ausführungsform werden die Alkohole $R^4OH$ bei erhöhter Temperatur und reduziertem Druck von 1000 bis 50 mbar destillativ abgetrennt.

In einer bevorzugten Ausführungsform beträgt zu Beginn der Reaktion das Verhältnis von Alkohol der Formel (II) zu Orthoester der Formel (III) höchstens 3 zu 1, mehr bevorzugt 2.5 zu 1 und besonders bevorzugt 2 zu 1.

In einer bevorzugten Ausführungsform wird der Alkohol der Formel (II) in zwei oder mehr Stufen zugegeben.

In einer bevorzugten Ausführungsform liegt die Reaktionstemperatur während der Umsetzung von Alkohol der Formel (II) mit Orthoester der Formel (III) bei 80 bis 230°C.

In einer bevorzugten Ausführungsform wird am Ende der Reaktion, d. h. nachdem etwa 80% der erwarteten Methanol- bzw. Ethanolmenge abdestilliert wurde ein steigender Temperaturgradient angelegt, um die Niedrigsieder zum größten Teil zu entfernen. Besonders bewährt haben sich Temperaturgradienten von 180 - 245°C Außentemperatur in einem Zeitraum von ½ bis zu 7 Stunden. Dabei kann zusätzlich noch bis zu 40% der bisher verwendeten Katalysatormenge (also z. B. Benzoesäure) zugegeben werden. Darüber hinaus ist auch das Anlegen eines leichten Stickstoffstroms oder das Anlegen eines reduzierten Drucks (50 - 1000 mbar) vorteilig um noch vorhandenen Niedrigsieder zu entfernen.

Das beschriebene Verfahren löst die Aufgabe vorliegender Erfindung ein Syntheseverfahren für Orthoester zu finden, nach dessen Durchführung keine anorganischen Katalysatoren bzw. Nebenprodukte entfernt werden müssen. Es ist daher möglich, auf eine Aufarbeitung zu verzichten, bei der die Ausbeute an isoliertem Produkt reduziert würde und Abfälle anfallen. Das Produkt fällt klar, homogen und salzfrei an.

Der Reaktionsfortschritt und Umsatz kann über die entstehende Alkoholmenge ($R^4$-OH) verfolgt werden. Die Dichten der entstehenden Alkohole entsprechen denen der erwarteten Alkohole.

Reaktionsfortschritt und Umsatz können auch über die Massenbilanz ermittelt werden. Die Massenbilanz der abdestillierten Alkoholmengen stimmt mit den erwarteten Werten überein: (Masse des eingesetzten Orthoesters) + (Masse des eingesetzten Glykols) + (Masse des eingesetzten Katalysators) - (Masse des abdestillierten Alkohols) = Masse des isolierten Glykolorthoesters.

Der Umsatz lässt sich ebenfalls mittels $^1$H-NMR verfolgen. Der mittels $^1$H-NMR bestimmte Umsatz weicht von dem über die abdestillierten Alkoholmenge bestimmten Umsatz unter 2% ab. Da die Alkylgruppen der einzelnen Orthoester bzw. die Protonen bei Orthoformaten überlagen und nur in Ausnahmefällen einzeln integriert werden können, wird die im folgenden abgeleitete Gleichung zur Umsatzbestimmung a ($0 \leq a \leq 1$) benutzt. Die Formel entspricht prinzipiell genau einer zur Bestimmung der Gleichgewichtskonstanten von Gasphasenreaktionen in Abhängigkeit des Dissoziationsgrades des betrachteten Edukts (siehe z. B.: Dickerson, Gray, Haight; Prinzipien der Chemie, Berlin/ New York 1978, S. 780) benutzten Formel.

Die untenstehende Tabelle soll die Ableitung verdeutlichen. Zu Beginn der Reaktion liegen das verwendete Glykol und Trimethylorthoacetat (1) im stöchiometrischen Mol-Verhältnis 3 : 1 vor. Zu einem beliebigen Zeitpunkt der

Reaktion hat sich der Anteil a (0≤ a ≤1) des Trimethylorthoacetat (1) umgesetzt, übrig sind also 1-a mol. Gleichzeitig haben sich - über alle Orthoacetate gemittelt - a mol Triglykolorthoacetat gebildet. Dabei wurden 3*a mol Glykol verbraucht, es liegen also noch 3-3*a mol Glykol vor. Gleichzeitig liegen noch 3*(1-a) $(H_3CO)$-C-$CH_3$ sowie a Me (im gebildeten Glykolorthoacetat) Gruppen vor (siehe untenstehende Tabelle).

| | Trimethylorthoacetat (1) | Glykol | Triglykolorthoacetat | Methanol |
|---|---|---|---|---|
| Start [mol] | 1 | 3 | 0 | 0 |
| Laufende Rkt. [mol] | 1-α | 3-3α | α | 3α |
| [mol] (MeO)C-Me Gruppen in 1 | 3-(1-α)[MeO] | | α [Me] | |

$$\int \frac{OMe}{Me} = \frac{3*(1-\text{alpha})}{alpha+(1-alpha)} = 3\cdot(1-\alpha)$$

Der Umsatz kann also aus den zusammen integrierten Methylprotonen (Me-C$(OR)_3$) aller Orthoester (d = 1.45 - 1.48 ppm) und den zusammen integrierten Me-C$(OMe)_n$ Protonen (d = 3.27 - 3.28 ppm) aller Orthoester bestimmt werden.

Für die entsprechenden Orthoformiate wird analog folgende Formel erhalten:

$$\int \frac{OMe}{H-C} = \frac{9*(1-\text{alpha})}{alpha+(1-alpha)} = 9\cdot(1-\alpha)$$

Der Umsatz kann also hier aus den zusammen integrierten Protonen (H-C$(OR)_3$) aller Orthoester und den zusammen integrierten H-C$(OMe)_n$ Protonen ($\delta$ = 3.27 - 3.28 ppm) aller Orthoester bestimmt werden.

Die Alkohole der Formel (II) sind durch literaturbekannte Alkoxylierungsverfahren zugänglich. Alkohole der Formel (II) können als Alkoxylierungsprodukte mit einer breiten Verteilung der verschiedenen Homologen mit Alkoxylierungsgrad m vorliegen, oder als durch Destillation getrennte Reinkomponenten, sowie Mischungen verschiedener Alkohole nach Formel (II). In einer Ausführungsform werden bevorzugt Mischungen von Alkoholen nach Formel (II) eingesetzt, die sich in $R_3$, $R^5$ und/oder m unterscheiden.

Bevorzugt werden durch Destillation gereinigten Alkoxylate mit m =1-6 verwendet, die einen geringen Alkaligehalt besitzen (Na/K < 50 ppm), sowie Mischungen solcher Alkohole.

Folgende Alkohole der Formel (II) wurden in den Synthesebeispielen verwendet und sind als bevorzugt anzusehen:

Methyltriglykol ($R^3$ = $CH_3$, m = 3, $R^5$ = H), bevorzugt in einer Reinheit von > 90% Methyltetraglykol ($R^3$ = $CH_3$, m = 4, $R^5$ = H), bevorzugt in einer Reinheit von > 85% Butyltri/Butyltetraglykol-Mischung ($R^3$ = n-Butyl), bevorzugt in einer Reinheit von > 90%, wobei insbesondere min. 60% mit m = 3 und min. 20% mit m = 4 vorliegen Dipropylenglykol-mono-Butylether ($R^3$ = n-Butyl, m = 2, $R^5$ = $CH_3$), bevorzugt in einer Reinheit von > 80% Polyglykol M 1250 ($R^3$ = $CH_3$, m = ca. 28, $R^5$ = H), erhältlich als Produkt der alkalischen Ethoxylierung von Methanol mit 28 Äquivalenten Ethylenoxid

Die verwendeten Orthoester-Ausgangmaterialien der Formel (III) sind kommerziell verfügbar und werden in der handelsüblichen Reinheit, in der Regel > 90% und ohne zusätzliche Vorbehandlung eingesetzt. Bevorzugte Ausgangsmaterialien sind Orthoester mit $R^4$ = $C_1$ bis $C_4$, da die resultierenden Alkohole während der Umsetzung leicht entfernt werden können. Bevorzugt werden Trimethylorthoformiat, Triethylorthoformiat, Trimethylorthoacetat sowie Triethylorthoacetat eingesetzt. Orthoester Formel (III) können auch nach literaturbekannten Verfahren durch die Umsetzung von Nitrilen und den jeweiligen Alkoholen oder Mischungen daraus hergestellt werden (siehe auch GB-1128963).

In einer Ausführungsform wird eine Mischung aus mindestens einem Alkylpolyalkylenglykol der Formel (II) und mindestens einem Polyalkylenglykol der Formel (V)

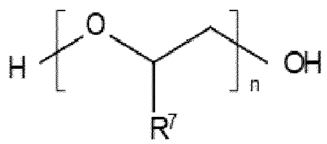

(V)

worin

n für eine Zahl von 1 bis 8 und
$R^7$ für Methyl oder H stehen

für die Umsetzung mit dem Orthoester der Formel (III) eingesetzt, um eine Mischung aus verbrückten Orthoestern der Formel (Id) sowie Orthoestern der Formel (I) zu erhalten

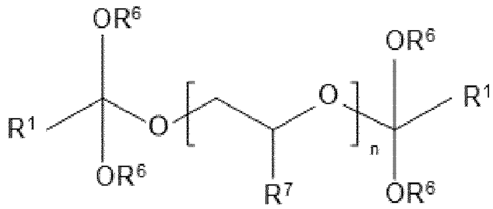
(Id)

worin

$R^1$ für H oder einen $C_1$- bis $C_8$-Alkylrest,
$R^6$ für einen $R^2$, oder eine Gruppe der Formel (VI)

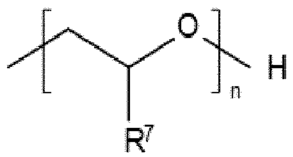
(VI)

$R^7$ für H oder Methyl,
n für eine Zahl von 1 bis 8 stehen, mit der Maßgabe, dass mindestens einer der Reste $R^2$ für eine Gruppe der Formel (IV) steht, und dass mindestens einer der Reste $R^6$ für $R^2$ steht.

Polyalkylenglykole der Formel (V) können entweder zu Beginn der Reaktion als Mischung mit Alkylpolyalkylenglykolen der Formel (II) eingesetzt werden, oder im Verlauf der Reaktion zwischen dem Orthoester der Formel (III) und den Alkylpolyalkylenglykolen der Formel (II) dem Reaktionsgemisch zugesetzt werden.
Die Verbrückung von Orthoester mittels Polyalkylenglykolen hat den technischen Effekt, die Viskosität der im erfindungsgemäßen Verfahren erhaltenen Produkte zu erhöhen, wobei ein höherer Anteil an Polyalkylenglykol der Formel (V) durch die Erzeugung eines größeren Anteil verbrückter Orthoester der Formel (Id) eine höhere Viskosität ergibt. Dabei werden Polykalkylenglykole der Formel (V) stets als Minderkomponente in Bezug auf die Alkylpolyalkylenglykole der Formel (II) eingesetzt, um die Bildung größerer Oligomere von Orthoestern und dreidimensionale Vernetzung, oder Verzweigung, zu vermeiden.
Polylalkylenglykole der Formel (V) werden bevorzugt in einer Menge von 0 bis 30 mol-%, bezogen auf die Menge an Alkylpolyalkylenglykole der Formel (II) als 100 mol-%, eingesetzt, mehr bevorzugt im Bereich von 0 bis 15 mol-%.
Die Reaktionsbedingungen und die Reaktionsführung in Anwesenheit von Polylalkylenglykolen der Formel (V) sind identisch zu den in Abwesenheit von Polylalkylenglykolen der Formel (V) beschriebenen.
Um das Entstehen verbrückter Orthoester der Formel (Id) zu erzielen ist es vorteilhaft, wenn die Gesamtmenge aus Alkylpolyalkylenglykolen der Formel (II) und Polylalkylenglykolen der Formel (V) ein molares Verhältnis zum Orthoester von 4 : 1, bevorzugt 3.5 : 1, nicht übersteigt. Ein größerer molarer Überschuss der Glykole führt verstärkt zur Bildung von nicht-verbrückten Orthoestern der Formel (I), bei denen $R^2$ entweder einen Alkylpolyalkylenglykolrest aus den Alkylpolyalkylenglykolen der Formel (II) oder einen Polyalkylenglykolrest aus den Polylalkylenglykolen der Formel (V) bedeutet.

Beispiele

Synthesebeispiel A - Trimethylorthoacetat + Methyltriglykol

In einem 1 Liter Vierhalskolben, ausgestattet mit einem Rührer und einem Destillationsaufsatz inkl. Vigreuxkolonne, wird unter Stickstoff eine Mischung aus 160.3 g (1.33 mol) Trimethylorthoacetat und 420 mL (2.66 Mol) Methyltriglykol vorgelegt und innerhalb von 40 min auf 165 °C erhitzt. Nach Erreichen der konstanten Reaktionstemperatur wurde während 60 min eine erste Teilmenge Methanol abdestilliert. Die Reaktionstemperatur wurde auf 185 °C erhöht

und für weitere 45 min gehalten, wobei insgesamt ca. 83% (71 g, 2.22 Mol) der theoretischen Methanol-Menge abdestilliert wurde. Danach wurden bei konstanter Heizleistung weitere 211 ml (1.34 Mol) Methyltriglykol zudosiert, und nach einer Stunde die Reaktionstemperatur auf 225 °C erhöht und für eine Stunde gehalten, wobei weiteres Methanol abdestilliert wurde. Danach wurden bei konstanter Heizleistung weitere 50 ml (0.32 Mol) Methyltriglykol zudosiert, sowie 1.9 g Benzoesäure zugegeben. Nach 3 Stunden wurden nochmals 0.9 g und nach noch einer weiteren Stunde schließlich nochmals 0.4 g Benzoesäure zugegeben. Abschließend wurde noch 2 h bei 235 °C Reaktionstemperatur gerührt. Es wurden insgesamt 140 ml/110.6 g Methanol (3.45 Mol) abdestilliert was einem Umsatz von 86.3% entspricht.

Der Orthoester wurde als klare, homogene, gelbliche Flüssigkeit in einer Menge von 727.6 g erhalten. Dies entspricht 98.7% bezogen auf die eingesetzte Masse und den Alkoholaustrag. Die Transparenz des Produkts wurde durch eine optische Trübungsmessung bestätigt. Durch die Menge an entferntem Methanol (3.45 mol) und der Startmenge Orthoester (1.33 mol) lässt sich berechnen, dass ca. 2.60 Äquivalente der Reste OR4 in Formel (III) durch alkoxylierte Alkohole der Formel (II) ersetzt wurden (3.45 mol).

Die Zusammensetzung des Produktes und der Abreaktionsgrad wurde ebenfalls mit $^1$H-NMR bestätigt: $^1$H-NMR ($CDCl_3$): $\delta$ = 1.48 (s, 3H, $H_3CC(OR)_3$), 3.38 (s, 9H, $OCH_3$), 3.55 ($m_c$, 6H, $H_3C\text{-}C(OCH_2\text{-}R)_3$), 3.6 - 3.75 ($m_c$, 30H, $OCH_2$). Der Umsatz laut NMR beträgt 88%

Nach analoger Anwendung der Vorschrift des Synthesebeispiels A wurden folgende Orthoester hergestellt. Die Synthesebeispiele B - D unterscheiden sich vom Synthesebeispiel A durch die Ansatzgröße und die Reaktionspartner der Formel (II) und (III). Die Menge an Benzoesäure als Katalysator war in den Beispielen B - C wie in A, ebenso die Temperaturen und Reaktionszeiten. Die beobachteten Ausbeuten sind in Tabelle 1 angegeben.

Die Synthesebeispiele D, E und F wurden unter Reaktionsbedingungen analog zu Beispiel A durchgeführt, mit dem Unterschied, dass 0.05 wt.-% 2-Ethylhexylphosphat als Katalysator verwendet wurde. Die Vergleichsbeispiele H und I stellen nicht erfindungsgemäße Synthesen dar, welche durch Einsatz eines unlöslichen, sauren Ionentauschers oder einer Lewis-Säure durchgeführt wurde.

| | |
|---|---|
| Synthesebeispiel B - | Trimethylorthoformiat + Methyltriglykol im molaren Verhältnis 1 : 3. |
| $^1$H-NMR ($CDCl_3$): $\delta$ = | 3.38, 3.39 (2s, 9H, $OCH_3$), 3.56 ($m_c$, 6H, $H\text{-}C(OCH_2\text{-}R)_3$), 3.6 - 3.74 ($m_c$, 30H, $OCH_2$), 5.31 (s, 1H, $HC(OR)_3$). |
| Synthesebeispiel C - | Trimethylorthoacetat + Mischung aus Methyltriglykol und Methyltetraglykol im molaren Verhältnis 1 : 1.5 zu 1.5 |
| $^1$H-NMR ($CDCl_3$): $\delta$ = | 1.48 (s, $H_3CC(OR)_3$), 3.38 (2 s, 2*$OCH_3$), 3.56 ($m_c$, $H_3C\text{-}C(OCH_2\text{-}R)_3$), 3.6 - 3.75 ($m_c$, $OCH_2$). |
| Synthesebeispiel D - | Triethylorthoacetat + Mischung aus Methyltriglykol, Butyltriglykol und Butyltetraglykol im molaren Verhältnis 1 : 1.5 zu 1.12 zu 0.38 |
| $^1$H-NMR ($CDCl_3$): $\delta$ = | 0.91 (t, propyl-$CH_3$), 1.34 - 1.39 ($m_c$, $CH_2CH_2\text{-}CH_3$), 1.48 (s, $H_3CC(OR)_3$), 1.54 - 1.58 ($m_c$, $CH_2\text{-}CH_2CH_3$), 3.38, (s, $OCH_3$), 3.46 ($m_c$, $H_3C\text{-}C(OCH_2\text{-}R)_3$), 3.56 - 3.74 ($m_c$, $OCH_2$). |
| Synthesebeispiel E - | Trimethylorthoacetat + Dipropylenglykolmonobutylether in molaren Verhältnis 1 zu 3.3. |
| Synthesebeispiel F - | Trimethylorthoacetat + Polyglykol M 1250 im molaren Verhältnis 1 zu 3.5. |
| | Synthesebeispiel G - Trimethylorthoacetat + Methyltriglykol + Diethylenglykol + Triethylenglykol im molaren Verhältnis 1 zu 3.08 zu 0.12 zu 0.08 (verbrückter Orthoester) |

In einem 1 Liter Vierhalskolben, ausgestattet mit einem Rührer und einem Destillationsaufsatz inkl. Vigreuxkolonne, wird unter Stickstoff eine Mischung aus 160.3 g (1.29 mol) Trimethylorthoacetat und 2/3 einer Glykolmischung - hergestellt aus 610.6 g (3.68 Mol) Methyltriglykol, 16.5 g Diethylenglykol (0.15 Mol) und 15.5 g (0.1 Mol) Triethylenglykol - vorgelegt und innerhalb von 90 min auf 150°C erhitzt. Nach Erreichen der konstanten Reaktionstemperatur wurde während 60 min eine erste Teilmenge Methanol abdestilliert. Die Reaktionstemperatur wurde auf 185 °C erhöht und für weitere 60 min gehalten, wobei insgesamt ca. 42% (51.9 g, 1.62 Mol) der theoretischen Methanol-Menge abdestilliert wurde. Danach wurden bei einer Reaktionstemperatur von 200 °C das restliche Drittel der oben hergestellten Glykolmischung zudosiert, sowie 1.7 g Benzoesäure zugegeben und nach einer Stunde nochmals 0.9 g Benzoesäure zugegeben und die Reaktionstemperatur nach einer weiteren Stunde auf 225 °C erhöht und für eine weitere Stunde gehalten, wobei weiteres Methanol abdestilliert wurde, insgesamt ca. 76% (94.5 g, 2.95 Mol) der theoretischen Menge. Bei konstanter Heizleistung werden weitere 50 g (0.3 Mol) Methyltriglykol zudosiert, sowie 0.7 g Benzoesäure zugegeben und weitere 7 Stunden bei 225 °C Reaktionstemperatur gerührt. Es wurden insgesamt 107.9 g Methanol (3.37 Mol) abdestilliert was einem Umsatz von 87% entspricht.

Der Orthoester wurde als klare, homogene, bernsteinfarbene Flüssigkeit in einer Menge von 723.2 g erhalten. Dies entspricht 98.1% bezogen auf die eingesetzte Masse und den Alkoholaustrag. Die Transparenz des Produkts wurde durch eine optische Trübungsmessung bestätigt.

Die Zusammensetzung des Produktes und der Abreaktionsgrad wurde ebenfalls mit $^1$H-NMR bestätigt: $^1$H-NMR ($CDCl_3$): $\delta$ = 1.48 (s, $H_3CC(OR)_3$), 3.38 (s, $OCH_3$), 3.55 ($m_c$, $H_3C-C(OCH_2-R)_3$), 3.6 - 3.75 ($m_c$, $OCH_2$).

Eine Unterscheidung zum im Synthesebeispiel A hergestellten Produkt aus Trimethylorthoacetat + Methyltriglykol ist nur durch die anderen Integralverhältnisse im $^1$H-NMR möglich. Der Umsatz laut NMR beträgt 86%.

Vergleichsbeispiel H - Triethylorthoacetat 110 g (0.66 mol) + Methyltriglykol im molaren Verhältnis 1 zu 3 mit saurem Ionenaustauscher Amberjet™ 1000 H (3.3 g). Das Produkt wurde durch Filtration vom Bodensatz an unlöslichem Ionentauscher befreit.

Vergleichsbeispiel I - Synthese analog Beispiel 1 aus DE-2062034

In einem 1 Liter Vierhalskolben, wird unter Stickstoff eine Mischung aus 160.3 g (1.33 mol) Trimethylorthoacetat, 630 mL (4.00 Mol) Methyltriglykol sowie 1.33 g Aluminiumchlorid vorgelegt und für 3 Stunden bei bis zu 140 °C unter Rückfluss gerührt. Anschließend wird über eine Kolonne 148 g Ethanol (81% der Theorie) abdestilliert, dabei wird die Temperatur bis auf 180 °C erhöht. Das nach dem Abkühlen trübe Produkt wird in Toluol gelöst und mit Wasser gewaschen und anschließend filtriert. Die organische Phase wird über Magnesiumsulfat getrocknet und das Toluol am Rotationsverdampfer entfernt. Der Rückstand entspricht ca. 64% der theoretischen Ausbeute. Die Ausbeute konnte nicht per NMR via obiger Formel bestimmt werden. Es ist davon auszugehen, dass Orthoester bevorzugt nach dem Literaturbekannten Verfahren bei Anwesenheit von Lewis-Säuren zur den entsprechenden Carbonsäureestern umgesetzt werden (vgl.
Houben-Weyl, Methoden der Organischen Chemie Vol. VI/3, Kapitel VIII, B. Umwandlung von Orthocarbonsäureestern, Seite 313ff).

Tabelle 1: Ausbeuten und Umsatz der Orthoester

| Synthesebeispiel | Isolierte Ausbeute (vs. Theorie)[1] | Umsatz (NMR)[2] | Esterbildung (NMR)[3] | Umsatz (Alkoholaustrag)[4] | Trübheit[5] [NFU] |
|---|---|---|---|---|---|
| A | 98.7% | 88% | 0.08 | 86.3% | 0.4 |
| B | 99.4% | 100% | 0 | 98% | 0.2 |
| C | 100% | 90% | 0.06 | 88% | 0.2 |
| D | 99.6% | 90% | 0.1 | 91% | 0.3 |
| E | 99.5% | 87% | 0.1 | 88% | 0.3 |
| F | 97.1% | 85% | 0.09 | 86% | 0.4 |
| G | 98.1% | 86% | 0.05 | 87% | 0.2 |
| Vgl. H | 100% | 90% | 7 | 87.6% | 1.6 |
| Vgl. I | 64% | n.b.[6] | n.b.[6] | 81% | 4.3 |

[1] isolierte, gewogene Produktausbeute in Prozent gegenüber der theoretischen Einsatzmenge, abzüglich entferntem Alkohol
[2] Umsatzberechnung - d.h. prozentualer Anteil der ausgetauschten MeOH bzw. EtOH Gruppen der eingesetzten Orthoester - nach $^1$H-NMR Signalen (Formel siehe oben). Ein kleinerer Wert als die isolierte Ausbeute ist also kein Widerspruch, weil auch nach der Reaktion noch EtO- bzw. MeO-Gruppen in den gebildeten Orthoestern vorhanden sind.
[3] Ratio Glykolester (Ameisensäure/Essigsäure)/ Orthoester bestimmt durch $^1$H-NMR
[4] Gravimetrische Umsatzbestimmung nach ausgetragenem Alkohol gegenüber der theoretischen Menge laut Einsatzmengen
[5] Trübungsmessung am Turbidimeter (Hach-Lange 2100N IS) der Rohprodukte (vor Aufarbeitung bei Vergleichsbeispiel I)
[6] nicht bestimmt, da eine Produktbildung nicht eindeutig nachgewiesen werden konnte.

## Patentansprüche

1. Verfahren zur Herstellung eines Orthoesters der Formel (I)

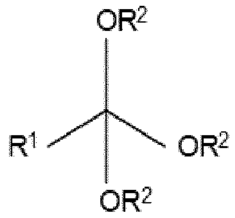 (I)

worin

$R^1$ für H oder einen $C_1$- bis $C_8$-Alkylrest,
$R^2$ für einen $C_1$- bis $C_6$-Alkylrest, oder eine Gruppe der Formel (IV)

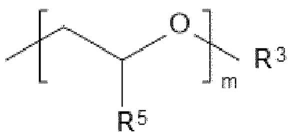 (IV)

$R^3$ für einen $C_1$- bis $C_6$-Alkylrest, und
m für eine Zahl von 1 bis 40 stehen, mit der Maßgabe, dass mindestens einer der Reste $R^2$ für eine Gruppe der Formel (IV), und
$R^5$ für H oder Methyl steht

indem mindestens ein Alkohol der Formel (II)

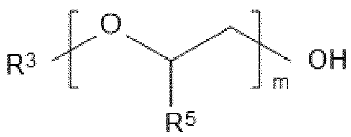 (II)

mit einem Orthoester der Formel (III)

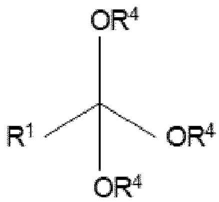 (III)

worin $R^4$ für eine $C_1$- bis $C_6$-Alkylgruppe steht
bei einer Temperatur von 60 bis 280°C umgesetzt wird, und die dabei entstehenden Alkohole $R^4$-OH destillativ entfernt werden, **dadurch gekennzeichnet, dass** die Umsetzung entweder in Abwesenheit eines Katalysators erfolgt, oder ein Katalysator, ausgewählt aus aromatischen Carbonsäuren und organischen Phosphorsäureestern zugegen ist.

**2.** Verfahren zur Herstellung eines Orthoesters nach Anspruch 1, wobei das molare Verhältnis der von Alkohol der Formel (II) zu Orthoester der Formel (III) mindestens 3 zu 1 beträgt.

**3.** Verfahren zur Herstellung eines Orthoesters nach Anspruch 1 und/oder 2, unter Verwendung eines Katalysators der eine aromatische Carbonsäure oder ein organischer Phosphorsäureester ist.

**4.** Verfahren nach Anspruch 3, worin die aromatische Carbonsäure Benzoesäure ist.

**5.** Verfahren nach Anspruch 3, worin der Katalysator ein Phosphorsäureester eines linearen oder verzweigten Alkohols mit 6 bis 18 Kohlenstoffatomen ist.

6. Verfahren nach einem oder mehreren der Ansprüche 1 - 4, worin die Alkohole $R^4$-OH über eine Kolonne abgetrennt werden.

7. Verfahren nach einem oder mehreren der Ansprüche 1 - 6, worin am Beginn der Umsetzung das molare Verhältnis von Alkohol der Formel (II) zu Orthoester der Formel (III) höchstens 3:1 beträgt.

8. Verfahren nach einem oder mehreren der Ansprüche 1 - 7, worin der Alkohol der Formel (II) in 2 oder mehr Stufen zugegeben wird.

9. Verfahren nach einem oder mehreren der Ansprüche 1 - 8, worin die Reaktionstemperatur 80 bis 230°C beträgt.

10. Verfahren nach einem oder mehreren der Ansprüche 1 - 9, worin der Alkohol $R^4$-OH bei reduziertem Druck entfernt wird.

11. Verfahren nach einem oder mehreren der Ansprüche 1 - 10, worin die Orthoester der Formel (I) den Formeln

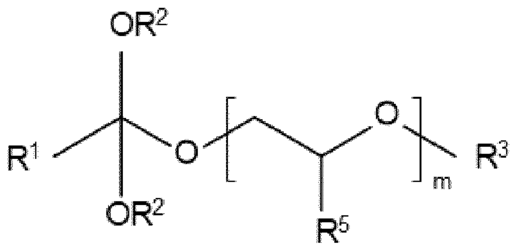

(Ia)

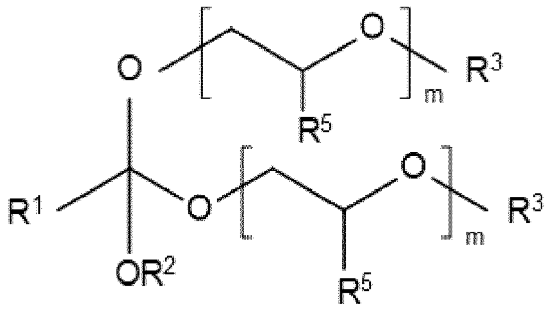

(Ib)

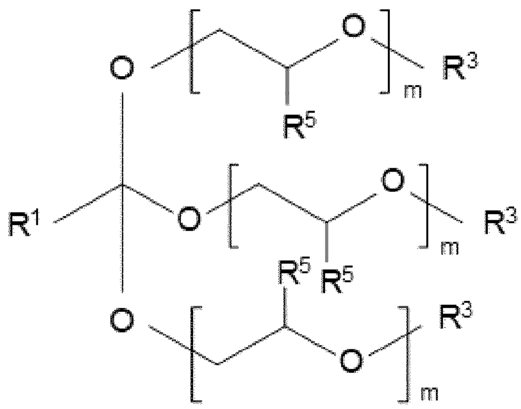

(Ic)

entsprechen.

12. Verfahren nach einem oder mehreren der Ansprüche 1 - 11, worin eine Mischung von Alkoholen der Formel (II) eingesetzt wird, die sich in $R^3$ und/oder m unterscheiden.

13. Verfahren nach einem oder mehreren der Ansprüche 1 - 12, worin eine Mischung aus verbrückten Orthoestern der Formel (Id) sowie Orthoestern der Formel (I)

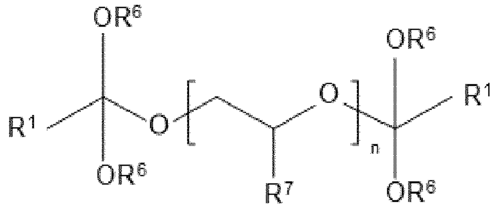

(Id)

hergestellt wird, worin

$R^6$ für $R^2$ oder eine Gruppe der Formel (VI)

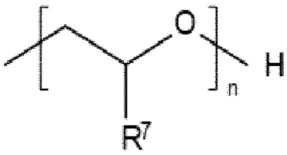

(VI)

$R^7$ für H oder Methyl, und
n für eine Zahl von 1 bis 8 stehen,

mit der Maßgabe, dass
mindestens einer der Reste $R^6$ für $R^2$ steht, und
mindestens einer der Reste $R^2$ für eine Gruppe der Formel (IV) steht,
indem neben mindestens einem Alkylpolyalkylenglykol der Formel (II) mindestens ein Polyalkylenglykol der Formel (V)

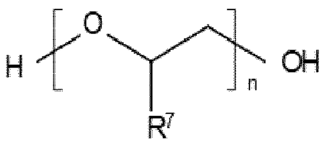

(V)

für die Umsetzung mit dem Orthoester der Formel (III) eingesetzt wird, und worin Polyalkylenglykole der Formel (V) in einer Menge von 0 bis 30 mol-%, bezogen auf die Menge an Alkylpolyalkylenglykolen der Formel (II) als 100 mol-%, eingesetzt werden.

**14.** Verfahren nach Anspruch 13, worin die Gesamtmenge aus Alkylpolyalkylenglykolen der Formel (II) und Polyalkylenglykolen der Formel (V) ein molares Verhältnis zum Orthoester der Formel (III) von 4 : 1 nicht übersteigt.

Europäisches Patentamt
European Patent Office
Office européen des brevets

# EUROPÄISCHER RECHERCHENBERICHT

Nummer der Anmeldung
EP 21 16 2203

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (IPC) |
| Y,D | DE 20 62 034 A1 (CASTROL LTD.)<br>1. Juli 1971 (1971-07-01)<br>* Beispiele 1-18 *<br>----- | 1-14 | INV.<br>C07C41/60<br>C07C43/32 |
| Y | BRACHVOGEL RENÉ-CHRIS ET AL: "Orthoester exchange: a tripodal tool for dynamic covalent and systems chemistry",<br>CHEMICAL SCIENCE,<br>Bd. 6, Nr. 2, 1. Januar 2015 (2015-01-01), Seiten 1399-1403, XP055834596,<br>United Kingdom<br>ISSN: 2041-6520, DOI: 10.1039/C4SC03528C<br>Gefunden im Internet:<br>URL:https://pubs.rsc.org/en/content/articlepdf/2015/sc/c4sc03528c><br>* Schema 2; Tabelle 1 *<br>----- | 1-14 | |
| Y | WO 99/32424 A1 (AKZO NOBEL NV [NL]; BERGSTROEM KARIN [SE]; HELLBERG PER ERIK [SE]) 1. Juli 1999 (1999-07-01)<br>* Beispiele 1-8 *<br>----- | 1-14 | RECHERCHIERTE SACHGEBIETE (IPC)<br>C07C |

Der vorliegende Recherchenbericht wurde für alle Patentansprüche erstellt

1

| Recherchenort | Abschlußdatum der Recherche | Prüfer |
|---|---|---|
| Den Haag | 25. August 2021 | Matés Valdivielso, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 21 16 2203

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

25-08-2021

| Im Recherchenbericht angeführtes Patentdokument | Datum der Veröffentlichung | Mitglied(er) der Patentfamilie | Datum der Veröffentlichung |
|---|---|---|---|
| DE 2062034 A1 | 01-07-1971 | AT 304743 B | 25-01-1973 |
| | | BE 760436 A | 27-05-1971 |
| | | CA 918679 A | 09-01-1973 |
| | | DE 2062034 A1 | 01-07-1971 |
| | | DK 138221 B | 31-07-1978 |
| | | FR 2073833 A5 | 01-10-1971 |
| | | GB 1330468 A | 19-09-1973 |
| | | IE 34803 B1 | 20-08-1975 |
| | | JP S4844230 B1 | 24-12-1973 |
| | | NL 147779 C | 25-08-2021 |
| | | NL 7018251 A | 18-06-1971 |
| | | ZA 708419 B | 26-07-1972 |
| WO 9932424 A1 | 01-07-1999 | AT 223367 T | 15-09-2002 |
| | | AU 739404 B2 | 11-10-2001 |
| | | BR 9813810 A | 03-10-2000 |
| | | CA 2310646 A1 | 01-07-1999 |
| | | CN 1282313 A | 31-01-2001 |
| | | DE 69807749 T2 | 08-05-2003 |
| | | EP 1042266 A1 | 11-10-2000 |
| | | JP 4386576 B2 | 16-12-2009 |
| | | JP 2001526250 A | 18-12-2001 |
| | | KR 20010033190 A | 25-04-2001 |
| | | US 2004039235 A1 | 26-02-2004 |
| | | WO 9932424 A1 | 01-07-1999 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 3419580 A
- GB 1128963 A
- DE 2062034
- US 2867667 A


### In der Beschreibung aufgeführte Nicht-Patentliteratur


- **R. M. ROBERTS ; T. D. HIGGINS ; P. R. NOYES.** *J. Am. Chem. Soc.,* 1955, vol. 77, 3801
- **E. R. ALEXANDER ; H. M. BUSCH.** *J. Am. Chem. Soc.,* 1952, vol. 74, 554
- **DICKERSON, GRAY, HAIGHT.** *Prinzipien der Chemie,* 1978, 780